# EUROPÄISCHE PATENTSCHRIFT

(11) **EP 3 523 270 B1**
(45) Veröffentlichungstag und Bekanntmachung des Hinweises auf die Patenterteilung: **21.10.2020**
(21) Anmeldenummer: 17780692.4
(22) Anmeldetag: 29.09.2017
(51) Int. Cl.: C07C 45/58, C07C 49/813, C07C 33/50, C07D 303/08, C07C 29/40, C07C 29/62

(54) **VERFAHREN ZUR HERSTELLUNG VON CYCLOPROPYL-SUBSTITUIERTEN ACETOPHENONEN**
VERFAHREN ZUR HERSTELLUNG VON CYCLOPROPYL-SUBSTITUIERTEN ACETOPHENONEN
PROCÉDÉ DE PRÉPARATION D'ACÉTOPHÉNONES SUBSTITUÉES PAR UN GROUPE CYCLOPROPYLE

(30) Priorität: 05.10.2016 EP 16192300
(43) Veröffentlichungstag der Anmeldung: 14.08.2019
(73) Patentinhaber: Bayer CropScience Aktiengesellschaft, 40789 Monheim am Rhein (DE)
(72) Erfinder: SCHOTES, Christoph, 40223 Düsseldorf (DE)
(74) Vertreter: BIP Patents
(86) Internationale Anmeldenummer: PCT/EP2017/074802
(87) Internationale Veröffentlichungsnummer: WO 2018/065316

(56) Entgegenhaltungen:
- CN-A- 101 857 576
- CN-B- 102 603 508

## Beschreibung

Die Erfindung betrifft ein Verfahren zur Herstellung von Cyclopropyl-substituierten Acetophenonen sowie zwei Verbindungen, die in dem Verfahren Verwendung finden.

Cyclopropyl-substituierte Acetophenone sind wichtige Intermediate zur Synthese von agrochemisch wirksamen Stoffen wie beispielsweise Cyproconazol und wurden berreits auf diversen Routen hergestellt. Als Beispiele seien hier zwei mit dem erfindungsgemäßen Verfahren verwandte Synthesen genannt:
CN 101857576A (2010) offenbart ein Verfahren, bei dem das Cyclopropyl-substituierte Acetophenon 1-(4-Chlorophenyl)-2-cycAlopropylpropan-1-on dargestellt wird durch Addition von 4-Chlorbenzylmagnesiumchlorid an Cyclopropylmethylketon, gefolgt von einer Eliminierung der entstandenen Hydroxygruppe, Hydroborierung und Oxidation zunächst zum benzylischen Alkohol, dann weiter zu 1-(4-Chlorophenyl)-2-cyclopropylpropan-1-on. Nachteilig an diesem Verfahren ist die Hydroborierungsreaktion, die sicherheitstechnisch bedenktlich und zudem teuer ist.A

CN102603508B beschreibt ebenfalls die Herstellung von 1-(4-Chlorophenyl)-2-cyclopropylpropan-1-on, jedoch ausgehend von Methyl 2-Chloro-2-(4-chlorophenyl)acetat. Dieses wird mit einer starken Base und Methylcyclopropylketon zu einem Glycidester umgesetzt, welcher nach Verseifung und Ansäuern zu 1-(4-Chlorophenyl)-2-cyclopropylpropan-1-on umlagert. Nachteilig ist hier der Einsatz von Methyl 2-chloro-2-(4-Chlorophenyl)acetate als Ausgangsmaterial, welches zunächst in einer mehrstufigen Synthese hergestellt werden muss und somit die Kosten des Verfahrens erhöht.

Aufgabe der vorliegenden Erfindung ist die Bereitstellung eines Verfahrens zur Herstellung von Cyclopropyl-substituierten Acetophenonen, welches die Nachteile der aus dem Stand der Technik bekannten Verfahren überwindet.

Diese Aufgabe ist durch ein Verfahren zur Herstellung von Cyclopropyl-substituierten Acetophenonen (I), 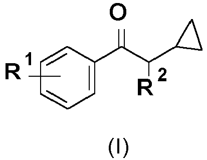 gelöst, bei dem
a) in einem ersten Schritt ein Benzylmagnesiumhalogenid oder Benzylzinkhalogenid (II) mit einem Cyclopropylalkylketon (III) zu einem Alkohol (IV) umgesetzt wird, 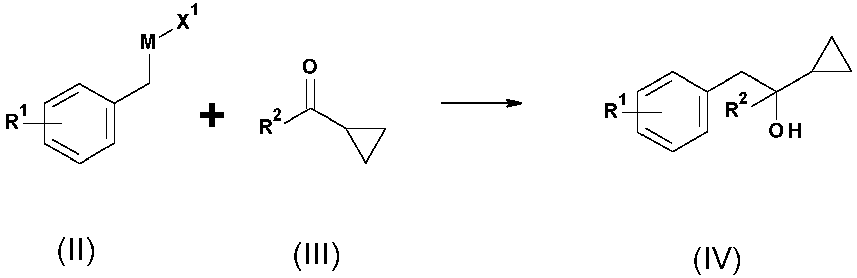
b) in einem zweiten Schritt der Alkohol (IV) durch radikalische Halogenierung zu einem Halogenhydrin (V) umgesetzt wird, 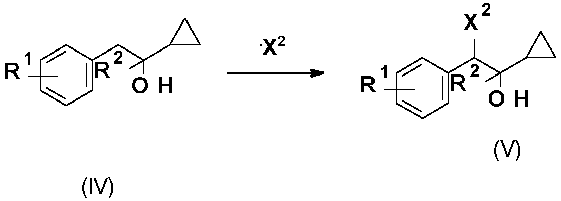
c) in einem dritten Schritt das Halogenhydrin (V) durch Zugabe von Base zu einem Epoxid (VI) umgesetzt wird, 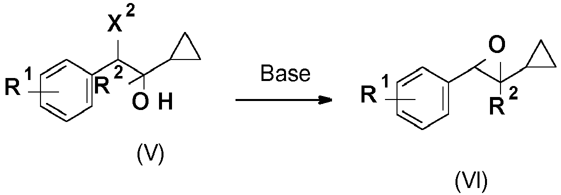
d) im einem vierten Schritt das Epoxid (VI) unter thermischen, Lewis-aziden oder Bronsted-aziden Bedingungen zu dem Cyclopropyl-substituierten Acetophenon (I) umgesetzt wird 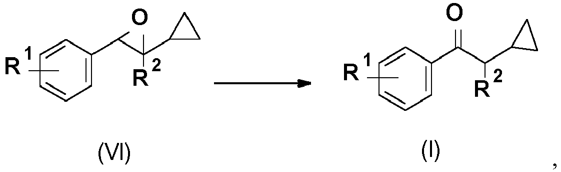 wobei
   - R¹: für Chlor, Brom, Iod, Alkoxy, Amino, Cyano, Nitro, C₁-C₄-Alkyl oder für unsubstituiertes oder durch s Reste aus der Gruppe bestehend aus Chlor, Fluor, Methoxy und Ethoxy substituiertes Phenyl steht,
   - R²: für C₁-C₄-Alkyl oder für unsubstituiertes oder durch s Reste aus der Gruppe bestehend aus Chlor, Fluor, Methoxy und Ethoxy substituiertes Pheny steht,
   s 1, 2 oder 3 ist,
   M Magnesium oder Zink ist.
   X¹,X² unabhängig voneinander Chlor, Brom oder Iod sind.

Gemäß einer bevorzugten Ausführungsform kann X¹ Chlor sein.

Bevorzugt ist ebenfalls, wenn X² Brom ist.

Ebenfalls bevorzugt ist, wenn R¹ 4-Chlor ist.

Ebenfalls bevorzugt ist, wenn M Magnesium ist.

Weiterhin bevorzugt ist, wenn R² Methyl ist.

Ganz besonders bevorzugt ist, wenn X¹ Chlor, X² Brom, R¹ 4-Chlor, M Magnesium, und R² Methyl sind.

In den Formeln (I), (II), (III), (IV), (V) und (VI) können Alkylreste mit mehr als zwei Kohlenstoffatomen geradkettig oder verzweigt sein. Geeignete Alkylreste sind z.B. Methyl, Ethyl, n- oder i-Propyl, n-, i-, t- oder 2-Butyl.

Verbindung (II) kann hergestellt werden, indem ein Benzylhalogenid mit Magnesium zum entsprechenden Benzylmagnesiumhalogenid umgesetzt wird, wobei ein Verhältnis von Magnesium zum Benzylhalogenid von 0,9:1 bis 20:1 eingesetzt werden kann.

Im ersten Schritt des erfindungsgemäßen Verfahrens wird das Benzylmagnesiumhalogenid der Formel (II) mit einem Cyclopropylalkylketon (III) zu einem Alkohol (IV) umgesetzt.

Die Reaktion wird in der Regel in einem Lösungsmittel durchgeführt. Geeignete Lösungsmittel sind aliphatische und aromatische Kohlenwasserstoffe, wie n-Hexan, Benzol oder Toluol und Ether wie Diphenylether, Methyl-tert-butylether, Isopropylethylether, Dioxan, Diglyme, Dimethylglycol, Dimethoxyethan und THF sowie Mischungen solcher Lösungsmittel. Bevorzugt sind Toluol/THF Mischungen sowie Diethylether. Besonders bevorzugt ist Diethylether.

Die Reaktion kann in einem Temperaturbereich von -40 bis 200 °C durchgeführt werden. Bevorzugt sind -20 bis 50°C, besonders bevorzugt 0 bis 40°C. Die Reaktion wird in der Regel bei Normaldruck durchgeführt, kann aber auch unter erhöhtem oder verringertem Druck durchgeführt werden.

Im zweiten Schritt des erfindungsgemäßen Verfahrens wird der Alkohol (IV) durch radikalische Halogenierung zu einem Halogenhydrin (V) umgesetzt.

Das für die Holgenierung verwendete Halogenradikal kann aus einem Halogenierungsreagenz unter Einwirkung von Hitze oder Strahlung (UV oder sichtbares Licht) und gegebenfalls eines Radikalstarters generiert werden.

Als Halogenierungsreagenz kommen beispielsweise folgende Substanzen in Frage: N-Chlorsuccinimid, N-Bromsuccinimid, N-Iodsuccinimid, Chlor, Brom, Iod, Bromtrichlormethan, 1,3-Dichloro-5,5-dimethylhydantoin, 1,3-Dibrom-5,5-dimethylhydantoin und Sulfurylchlorid. Bevorzugt ist N-Bromsuccinimid. Das Verhältnis der Verbindung der Formel (IV) zum Halogenierungsreagenz liegt bevorzugt zwischen 1:0,9 und 1:10.

Als Radikalstarter sind besonders Dibenzoylperoxid, 2,2'-Azobis(2-methylpropionitril) (AIBN) und Ditert-butylperoxid geeignet, welche in einer Menge von 0.1 bis 50 Molprozent, bezogen auf die Verbindung der allgemeinen Formel (IV), eingesetzt werden können. Bevorzugt werden 1 bis 20 Molprozent des Radikalstarters eingesetzt.

Die Reaktionstemperatur im zweiten Schritt des erfindungsgemäßen Verfahrens kann zwischen 0 und 150 °C betragen. Bevorzugt sind 20 bis 90 °C, besonders bevorzugt 60 bis 80 °C. Die Reaktion wird in der Regel bei Normaldruck durchgeführt, kann aber auch unter erhöhtem oder verringertem Druck durchgeführt werden.

Als Verdünnungsmittel können bei diesem Schritt im Prinzip alle unter den Reaktionsbedingungen inerten organischen Lösungsmittel verwendet werden. Beispielhaft seien genannt: Ether wie Methyl-tert-butylether, Methyl-cyclopentylether, 2-Methyltetrahydrofuran, 1,4-Dioxan; Kohlenwasserstoffe wie Cyclohexan, Methylcyclohexan, Toluol, Xylole, Mesitylen, Chlorbenzol, 1,2-Dichlorbenzol, Dichlormethan, Tetrachlormethan, Chloroform; Nitrile wie Acetonitril, Butyronitril; Amide wie N,N-Dimethylformamid, N,N-Dimethylacetamid, N-Methylpyrrolidon; Dimethylsulfoxid oder Sulfolan.

Im dritten Schritt des erfindungsgemäßen Verfahrens wird das Halogenhydrin (V) durch Zugabe von Base zu einem Epoxid (VI) umgesetzt.

Die Reaktion kann in Gegenwart von anorganischen Basen, wie LiOH, NaOH, KOH, Ca(OH)₂, Na₂CO₃, K₂CO₃, Cs₂CO₃, NaOMe, NaOEt, NaO-*t*-Bu, KO-*t*-Bu oder organischen Basen wie Trialkylaminen, Alkylpyridinen, Phosphazenen und 1,8-Diazabicyclo[5.4.0]undecen (DBU) durchgeführt werden. Bevorzugt sind NaOH und KOtBu, besonders bevorzugt KO-t-Bu.

Die Basen können in einem Mengenverhältnis von 1:0,9 bis 1:20 bezogen auf Verbindung (V) eingesetzt werden.

Wenn die Basen als wässrige Lösungen verwendet werden, wird zusätzlich ein Phasentransferkatalysator zugesetzt wie beispielweise Ammonium- oder Phosphonium-Salze, wie Tetrahexylammoniumchlorid, Tetrahexylammoniumbromid, Tetrahexyl-ammoniumiodid, Tetraoctylammoniumchlorid, Tetraoctylammoniumbromid, Tetraoctylammoniumiodid, Aliquat HTA-1®, Aliquat 134®, Dimethyldidecyl-ammoniumchlorid, Dimethyldodecylbenzylammoniumchlorid, Tributylhexadecyl-ammoniumchlorid, Tributylhexadecylammoniumbromid, Tributyltetradecylphosphoniumchlorid und Tributyltetradecylphosphoniumbromid. Bevorzugt ist Aliquat 134®.

Der Phasentransferkatalysator wird üblicherweise in einer Menge von 0,1 bis 50 Molprozent, bezogen auf die Verbindung (V) eingesetzt. Bevorzugt sind 5 bis 20 Molprozent.

Als Verdünnungsmittel können bei dieser Reaktion im Prinzip alle unter den Reaktionsbedingungen inerten organischen Lösungsmittel verwendet werden. Beispielhaft seien genannt: Ether wie Methyl-tert-butylether, Diethylether, Tetrahydrofuran, Methyl-cyclopentylether, 2-Methyltetrahydrofuran, 1,4-Dioxan; Kohlenwasserstoffe wie Cyclohexan, Methylcyclohexan, Toluol, Xylole, Mesitylen, Chlorbenzol, 1,2-Dichlorbenzol, Dichlormethan; Nitrile wie Acetonitril, Butyronitril; Amide wie N,N-Dimethylformamid, N,N-Dimethylacetamid, N-Methyl-pyrrolidon; Dimethylsulfoxid oder Sulfolan. Bevozugt sind THF, Toluol und Chlorbenzol.

Die Reaktion im dritten Schritt des erfindungsgemäßen Verfahrens wird in der Regel bei einer Temperatur von -20 bis 70 °C, vorzugsweise 0 bis 20 °C, durchgeführt. Die Reaktion wird in der Regel bei Normaldruck durchgeführt, kann aber auch unter erhöhtem oder verringertem Druck durchgeführt werden.

Im vierten Schritt des erfindungsgemäßen Verfahrens wird das Epoxid (VI) unter thermischen, Lewis-aziden oder Brønsted-aziden Bedingungen zu dem Cyclopropyl-substituierten Acetophenon (I) umgesetzt.

Als Lewis-Säuren sind beispielsweise zu nennen: Bortrifluorid, Aluminiumtrichlorid, Zinktriflat, Scandiumtriflat, Zinkchlorid, Zinkbromid, Kupfer(II)chlorid, Titantetrachlorid, Trimethylsillylchlorid, Zinntetrachlorid, Certrichlorid, Magnesiumchlorid, Eisendichlorid und Eisentrichlorid. Besonders geeignet sind Certrichlorid, Zinktriflat, Zinkchlorid und Scandiumtriflat.

Als Brønsted Säuren kommen mineralische Säuren, wie H₂SO₄, HCl, HSO₃Cl, HF, HBr, HI, H₃PO₄ oder organischen Säuren wie CF₃COOH, *p*-Toluolsulfonsäure, Methansulfonsäure, Camphersulfonsäure oder Trifluormethansulfonsäure in Frage. Besonders geeignet sind Camphersulfonsäure und Methansulfonsäure.

Die Brønsted - und Lewis Säuren können in einem Mengenverhältnis von 0,1 bis 50 mol%, bezogen auf Verbindung (VI) zugesetzt werden. Bevorzugt sind 5 bis 30 mol%, besonders bevorzugt 10 bis 20 mol%.

Die Reaktion im vierten Schritt des erfindungsgemäßen Verfahrens wird in der Regel in einem Lösungsmittel durchgeführt.

Geeignete Lösungsmittel sind aliphatische und aromatische Kohlenwasserstoffe, wie n-Hexan, Benzol oder Toluol, welche mit Heteroatomen wie Fluor oder Chlor substituiert sein können, wie Dichlormethan, Dichlorethan, Chlorbenzol oder Dichlorbenzol. Ebenfalls in Frage kommen Ether wie Diphenylether, Methyl-tert-butylether, Isopropylethylether, Dioxan, Diglyme, Dimethylglycol, Dimethoxyethan und THF und Mischungen solcher Lösungsmittel. Bevorzugt sind Methyl-tert-butylether, Toluol und Chlorbenzol. Besonders bevorzugt sind Toluol und Chlorbenzol.

Die Reaktionstemperatur beträgt zwischen 0 und 100 °C, bevorzugt 20 bis 80 °C. Die Reaktion wird in der Regel bei Normaldruck durchgeführt, kann aber auch unter erhöhtem oder verringertem Druck durchgeführt werden.

Ebenfalls Gegenstand der Erfindung ist eine Verbindung der allgemeinen Formel (V), 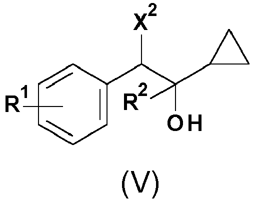 wobei
- R¹: für Chlor, Brom, Iod, Alkoxy, Amino, Cyano, Nitro, C₁-C₄-Alkyl oder für unsubstituiertes oder durch s Reste aus der Gruppe bestehend aus Chlor, Fluor, Methoxy und Ethoxy substituiertes Phenyl steht,
- R²: für C₁-C₄-Alkyl oder für unsubstituiertes oder durch s Reste aus der Gruppe bestehend aus Chlor, Fluor, Methoxy und Ethoxy substituiertes Pheny steht,
- s: 1, 2 oder 3 ist,
- X²: Chlor, Brom oder Iod ist.

Gemäß einer bevorzugten Ausführungsform der Verbindungen der Formel (V) kann X² Brom sein.

Bevorzugt ist, wenn R¹ 4-Chlor ist.

Weiterhin bevorzugt ist, wenn R² Methyl ist.

Ganz besonders bevorzugt ist, wenn X² Brom, R¹ 4-Chlor und R² Methyl sind.

Ebenfalls Gegenstand der Erfindung ist eine Verbindung der allgemeinen Formel (VI)), 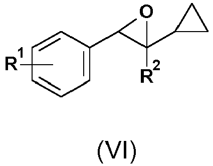 wobei
- R¹: für Chlor, Brom, Iod, Alkoxy, Amino, Cyano, Nitro, C₁-C₄-Alkyl oder für unsubstituiertes oder durch s Reste aus der Gruppe bestehend aus Chlor, Fluor, Methoxy und Ethoxy substituiertes Phenyl steht,
- R²: für C₁-C₄-Alkyl oder für unsubstituiertes oder durch s Reste aus der Gruppe bestehend aus Chlor, Fluor, Methoxy und Ethoxy substituiertes Pheny steht,
- s: 1, 2 oder 3 ist.

Gemäß einer bevorzugten Ausführungsform der Verbindungen der Formel (VI) kann R¹ 4-Chlor sein.

Ebenfalls bevorzugt ist, wenn R² Methyl ist.

Ganz besonders bevorzugt ist, wenn R¹ 4-Chlor und R² Methyl sind.

Die nachfolgenden Beispiele erläutern die Erfindung näher.

### Herstellung von 1-(4-Chlorophenyl)-2-cyclopropylpropan-1-one

### Schritt 1: 1-(4-Chlorophenyl)-2-cyclopropyl-propan-2-ol

Magnesiumspäne (30,4 g, 1,25 mol, 2 equiv) wurden mit einer Spur Iod in 300 mL Diethylether vorgelegt. 4-Chlorbenzylchlorid (100,6 g, 625 mmol, 1 equiv) wurde in 200 mL Diethylether gelöst und zum Magnesium zugetropft. Nach Ende der Zugabe wurde das Raktionsgemisch für 10 min bei Rückfluss gehalten. Es wurde auf 0 °C abgekühlt und Cyclopropylmethylketon wurde in 100 mL Diethylether so zugetropft, dass die Innentemperatur 10 °C nicht überschreitete. Anschließend wurde für 2 h bei Raumtemperatur nachgerührt. Das Reaktionsgemisch wurde mit 80 mL gesättigter Ammoniumchloridlösung gequenched und mit 50 mL Wasser versetzt. Die entstandene Suspension wurde über Kieselgur abfiltriert und mit Methyl-tert-butylether nachgespült. Die organische Phase wurde abgetrennt und mit Wasser und Brine gewaschen, und dann über Natriumsulfat getrocknet, gefiltert und einrotiert. Es wurden 122 g 1-(4-chlorophenyl)-2-cyclopropyl-propan-2-ol mit einer Reniheit von 100% (GCa/a) erhalten (85% Ausbeute).
¹H NMR (602MHz, DMSO-d₆) δ = 7.32 - 7.23 (m, 4H), 3.98 (s, 1H), 2.72 - 2.65 (m, 2H), 0.96 (s, 3H), 0.76 (tt, *J*=5.4, 8.4 Hz, 1H), 0.34 - 0.23 (m, 2H), 0.21 - 0.15 (m, 2H). GC/MS: m/e = 192 (M-H₂O).

### Schritt 2: 1-Bromo-1-(4-chlorophenyl)-2-cyclopropyl-propan-2-ol

1-(4-chlorophenyl)-2-cyclopropyl-propan-2-ol (20 g, 87,1 mmol, 1 equiv) wurde in Chlorbenzol (200 mL) zusammen mit N-Bromsuccinimid (26,54 g, 148 mmol, 1,7 equiv) und 2,2'-Azobis(2-methylpropionitril) (0,85 g, 3,5 mmol, 0,04 equiv) bei 70 °C für 44 h gerührt. Das Reaktionsgemisch wurde über Kieselgel filtriert und mit Chlorbenzol nachgewaschen. Nach Entfernen des Lösungsmittels ergab sich ein Rohprodukt von 27,6 g (59% Ausbeute, Reinheit 79,3%a/a HPLC), welches sich durch Säulenchromatographie (Kieselgel, Cyclohexan/Essigester-Gradient 0-20%v/v) auf eine Reinheit von 96,6%a/a HPLC aufreinigen lies.
Diastereoisomer A: ¹H NMR (602MHz, DMSO-d₆) δ = 7.56 - 7.50 (m, 2H), 7.41 - 7.36 (m, 2H), 5.21 (s, 1H), 4.70 (s, 1H), 1.27 (s, 1H), 0.87 (tt, *J*=5.4, 8.4 Hz, 1H), 0.51 - 0.43 (m, 1H), 0.32 - 0.13 (m, 2H), 0.08 - 0.02 (m, 1H). Diastereoisomer B: ¹H NMR (602MHz, DMSO-d₆) δ = 7.56 - 7.50 (m, 2H), 7.36 - 7.32 (m, 2H), 5.16 (s, 1H), 4.65 (s, 1H), 1.12 (s, 3H), 0.79 - 0.73 (m, 1H), 0.32 - 0.13 (m, 3H), -0.04 - - 0.10 (m, 1H). GC/MS: m/e = 208 (M-HBr).

### Schritt 3: 3-(4-Chlorophenyl)-2-cyclopropyl-2-methyl-oxiran

1-Bromo-1-(4-chlorophenyl)-2-cyclopropyl-propan-2-ol (5 g, 15,7 mmol, 1 equiv) wurde in 50 mL Toluol vorgelegt und auf 0 °C abgekühlt. Dann wurde Kalium-tert-butylat (2,7 g, 23,6 mmol, 1,5 equiv) zugegeben. Man lies sich das Reaktionsgemisch auf Raumtemperatur erwärmen und rührte für 2,5 Stunden bei dieser Temperatur nach. Das Reaktionsgemsich wurde über Kieselgur filtriert und direkt für den 4. Schritt weiter verwendet.
Diastereoisomer A: ¹H NMR (602MHz, DMSO-d₆) δ = 7.44 - 7.39 (m, 2H), 7.38 - 7.34 (m, 2H), 3.99 (s, 1H), 1.22 (tt, *J*=5.2, 8.3 Hz, 1H), 0.56 - 0.22 (m, 4H). Diastereoisomer B: ¹B NMR (602MHz, DMSO-d₆) δ = 7.44 - 7.39 (m, 2H), 7.31 - 7.27 (m, 2H), 3.87 (s, 1H), 1.01 (s, 3H), 0.54 - 0.23 (m, 5H). GC/MS: m/e = 208 (M).

### Schritt 4: 1-(4-Chlorophenyl)-2-cyclopropylpropan-1-one

Die toluolische Lösung aus Schritt 3 wurde mit Zinkchlorid (0,43 g, 3,16 mmol, 0,2 equiv) versetzt und für 15 h bei Raumtemperatur gerührt. Die entstandene Suspension wurde über Kieselgel filtriert und das Lösungsmittel unter reduziertem Druck entfernt. Man erhielt 3,9 g 1-(4-Chlorophenyl)-2-cyclopropylpropan-1-on als Rohprodukt, welches sich durch Säulenchromatographie (Kieselgel, Cyclohexan/Essigester-Gradient 0-20%v/v) auf eine Reinheit von 94%a/a (HPLCa/a) aufreinigen lies (2,02 g 55% Ausbeute über 2 Schritte).
¹H NMR (602MHz, DMSO-d₆) δ = 7.98 - 7.94 (m, 2H), 7.60 - 7.57 (m, 2H), 2.96 (qd, *J*=6.9, 9.1 Hz, 1H), 1.15 (d, *J*=6.9 Hz, 3H), 0.93 - 0.85 (m, 1H), 0.48 (ddt, *J*=3.9, 5.3, 8.7 Hz, 1H), 0.40 - 0.33 (m, 1H), 0.26 - 0.13 (m, 2H). GC/MS: m/e = 208 (M).

## Patentansprüche

1. Verfahren zur Herstellung von Cyclopropyl-substituierten Acetophenonen (I), 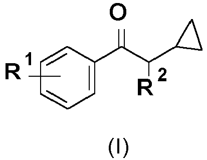 bei dem
a) in einem ersten Schritt ein Benzylmagnesiumhalogenid oder Benzylzinkhalogenid (II) mit einem Cyclopropylalkylketon (III) zu einem Alkohol (IV) umgesetzt wird, 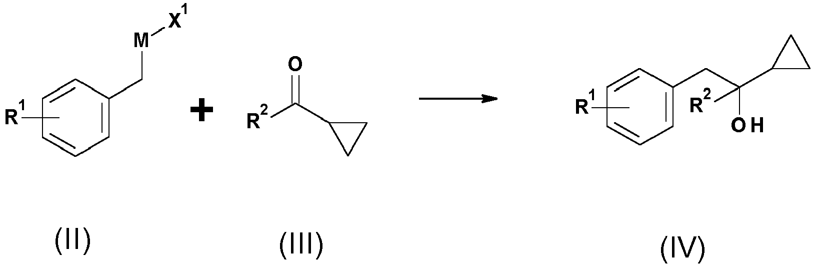
b) in einem zweiten Schritt der Alkohol (IV) durch radikalische Halogenierung zu einem Halogenhydrin (V) umgesetzt wird, 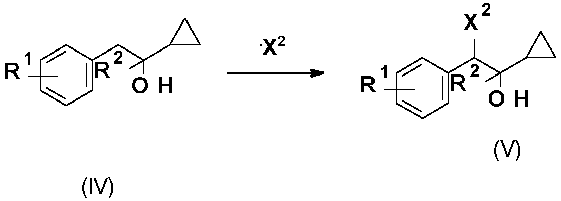
c) in einem dritten Schritt das Halogenhydrin (V) durch Zugabe von Base zu einem Epoxid (VI) umgesetzt wird, 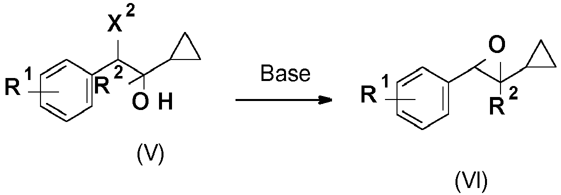
d) im einem vierten Schritt das Epoxid (VI) unter thermischen, Lewis-aziden oder Brønsted-aziden Bedingungen zu dem Cyclopropyl-substituierten Acetophenon (I) umgesetzt wird, 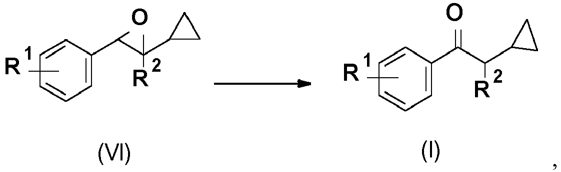 wobei
R¹ für Chlor, Brom, Iod, Alkoxy, Amino, Cyano, Nitro, C₁-C₄-Alkyl oder für unsubstituiertes oder durch s Reste aus der Gruppe bestehend aus Chlor, Fluor, Methoxy und Ethoxy substituiertes Phenyl steht,
R² für C₁-C₄-Alkyl oder für unsubstituiertes oder durch s Reste aus der Gruppe bestehend aus Chlor, Fluor, Methoxy und Ethoxy substituiertes Pheny steht,
s 1, 2 oder 3 ist,
X¹,X² unabhängig voneinander Chlor, Brom oder Iod sind.

2. Verfahren nach Anspruch 1, **dadurch gekennzeichnet, dass** X₁ Chlor ist.

3. Verfahren nach einem der Ansprüche 1 oder 2, **dadurch gekennzeichnet, dass** X² Brom ist.

4. Verfahren nach einem der Ansprüche 1 bis 3, **dadurch gekennzeichnet, dass**, R₁ 4-Chlor ist.

5. Verfahren nach einem der Ansprüche 1 bis 4, **dadurch gekennzeichnet, dass** und R₂ Methyl ist.

6. Verfahren nach Anspruch 1, bei dem X¹ Chlor, X² Brom, R¹ 4-Chlor und R₂ Methyl sind.

7. Verbindung der Formel (V), 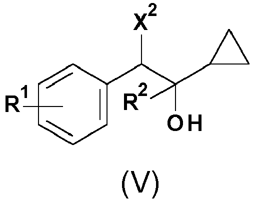 wobei
R¹ für Chlor, Brom, Iod, Alkoxy, Amino, Cyano, Nitro, C₁-C₄-Alkyl oder für unsubstituiertes oder durch s Reste aus der Gruppe bestehend aus Chlor, Fluor, Methoxy und Ethoxy substituiertes Phenyl steht,
R₂ für C₁-C₄-Alkyl oder für unsubstituiertes oder durch s Reste aus der Gruppe bestehend aus Chlor, Fluor, Methoxy und Ethoxy substituiertes Pheny steht,
s 1, 2 oder 3 ist,
X² Chlor, Brom oder Iod ist.

8. Verbindung nach Anspruch 7, bei der X² Brom, R¹ 4-Chlor und R₂ Methyl sind.

9. Verbindung der Formel (VI), 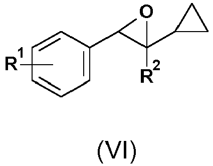 wobei
R¹ für Chlor, Brom, Iod, Alkoxy, Amino, Cyano, Nitro, C₁-C₄-Alkyl oder für unsubstituiertes oder durch s Reste aus der Gruppe bestehend aus Chlor, Fluor, Methoxy und Ethoxy substituiertes Phenyl steht,
R₂ für C₁-C₄-Alkyl oder für unsubstituiertes oder durch s Reste aus der Gruppe bestehend aus Chlor, Fluor, Methoxy und Ethoxy substituiertes Pheny steht,
s 1, 2 oder 3 ist.

10. Verbindung nach Anspruch 9, bei der R¹ 4-Chlor und R₂ Methyl sind.

## Claims

1. Method for preparing cyclopropyl-substituted acetophenones (I), 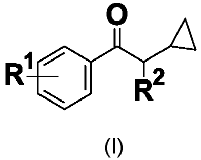 in which
a) in a first step a benzylmagnesium halide or benzylzinc halide (II) is reacted with a cyclopropyl alkyl ketone (III) to give an alcohol (IV), 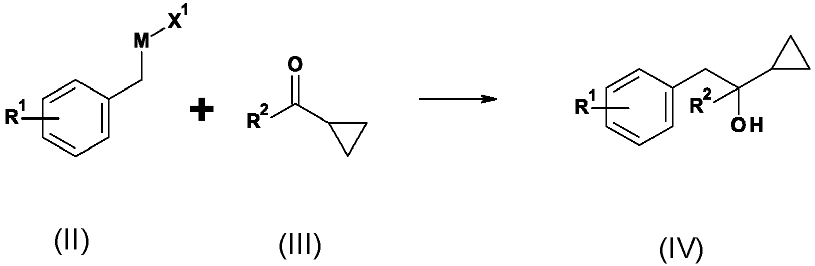
b) in a second step the alcohol (IV) is converted to a halohydrin (V) by free-radical halogenation, 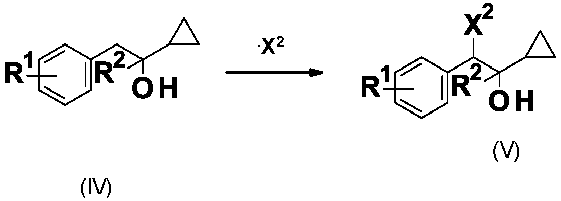
c) in a third step the halohydrin (V) is converted to an epoxide (VI) by addition of base, 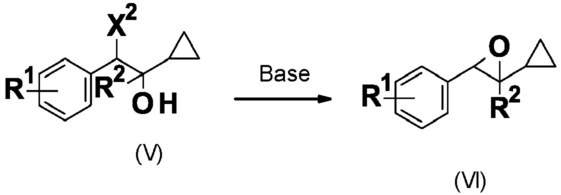
d) in a fourth step the epoxide (VI) is converted to the cyclopropyl-substituted acetophenone (I) under thermal Lewis acidic or Brønsted acidic conditions, 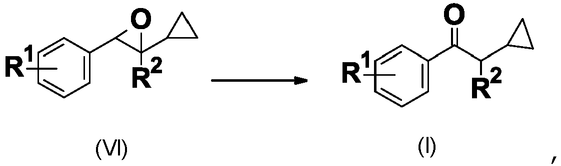 where
R¹ is chlorine, bromine, iodine, alkoxy, amino, cyano, nitro, C₁-C₄-alkyl or is unsubstituted phenyl or phenyl substituted by s radicals selected from the group consisting of chlorine, fluorine, methoxy and ethoxy,
R₂ is C₁-C₄-alkyl or is unsubstituted phenyl or phenyl substituted by s radicals selected from the group consisting of chlorine, fluorine, methoxy and ethoxy,
s is 1, 2 or 3,
X¹,X² are each independently chlorine, bromine or iodine.

2. Method according to Claim 1, **characterized in that** X¹ is chlorine.

3. Method according to either of Claims 1 and 2, **characterized in that** X² is bromine.

4. Method according to any of Claims 1 to 3, **characterized in that** R¹ is 4-chlorine.

5. Method according to any of Claims 1 to 4, **characterized in that** R₂ is methyl.

6. Method according to Claim 1, in which X¹ is chlorine, X² is bromine, R¹ is 4-chlorine and R₂ is methyl.

7. Compound of the formula (V), 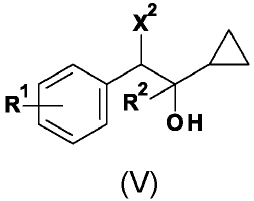 where
R¹ is chlorine, bromine, iodine, alkoxy, amino, cyano, nitro, C₁-C₄-alkyl or is unsubstituted phenyl or phenyl substituted by s radicals selected from the group consisting of chlorine, fluorine, methoxy and ethoxy,
R₂ is C₁-C₄-alkyl or is unsubstituted phenyl or phenyl substituted by s radicals selected from the group consisting of chlorine, fluorine, methoxy and ethoxy,
s is 1, 2 or 3,
X² is chlorine, bromine or iodine.

8. Compound according to Claim 7, in which X² is bromine, R¹ is 4-chlorine and R₂ is methyl.

9. Compound of the formula (VI), 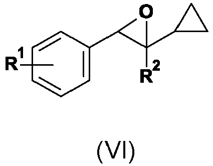 where
R¹ is chlorine, bromine, iodine, alkoxy, amino, cyano, nitro, C₁-C₄-alkyl or is unsubstituted phenyl or phenyl substituted by s radicals selected from the group consisting of chlorine, fluorine, methoxy and ethoxy,
R₂ is C₁-C₄-alkyl or is unsubstituted phenyl or phenyl substituted by s radicals selected from the group consisting of chlorine, fluorine, methoxy and ethoxy,
s is 1, 2 or 3.

10. Compound according to Claim 9, in which R¹ is 4-chlorine and R₂ is methyl.

## Revendications

1. Procédé pour la préparation d'acétophénones (I) substituées par cyclopropyle, 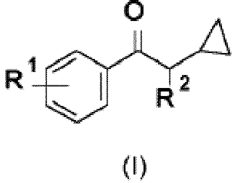 dans lequel
a) dans une première étape, un halogénure de benzylmagnésium ou un halogénure de benzylzinc (II) est transformé avec une cyclopropylalkylcétone (III) en un alcool (IV), 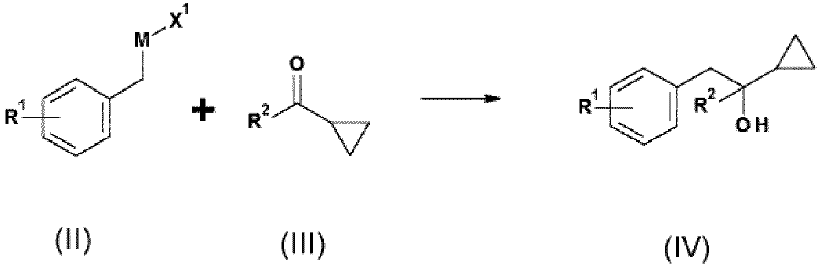
b) dans une deuxième étape, l'alcool (IV) est transformé par halogénation radicalaire en une halogénohydrine (V), 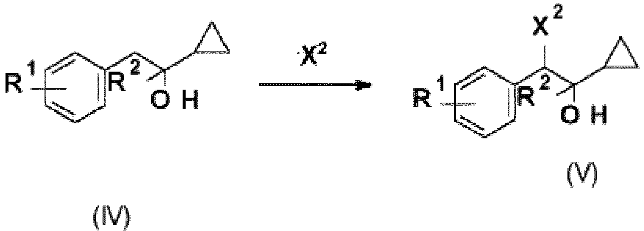
c) dans une troisième étape, l'halogénohydrine (V) est transformée par l'ajout d'une base en un époxyde (VI), 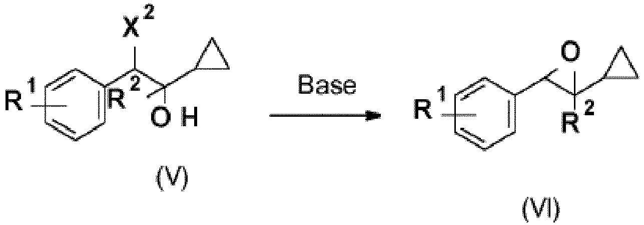
d) dans une quatrième étape, l'époxyde (VI) est transformé dans des conditions thermiques, d'azide de Lewis ou d'azide de Brønsted en l'acétophénone (I) substituée par cyclopropyle, 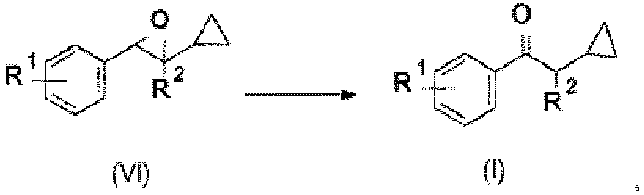
R¹ représentant chlore, brome, iode, alcoxy, amino, cyano, nitro, C₁-C₄-alkyle ou phényle non substitué ou substitué par s radicaux du groupe constitué par chlore, fluor, méthoxy et éthoxy,
R₂ représentant C₁-C₄-alkyle ou phényle non substitué ou substitué par s radicaux du groupe constitué par chlore, fluor, méthoxy et éthoxy,
s étant 1, 2 ou 3,
X¹, X² étant indépendamment l'un de l'autre chlore, brome ou iode.

2. Procédé selon la revendication 1, **caractérisé en ce que** X¹ est chlore.

3. Procédé selon l'une quelconque des revendications 1 et 2, **caractérisé en ce que** X² est brome.

4. Procédé selon l'une quelconque des revendications 1 à 3, **caractérisé en ce que** R¹ est 4-chloro.

5. Procédé selon l'une quelconque des revendications 1 à 4, **caractérisé en ce que** R₂ est méthyle.

6. Procédé selon la revendication 1, dans lequel X¹ est chlore, X² est brome, R¹ est 4-chloro et R₂ est méthyle.

7. Composé de formule (V), 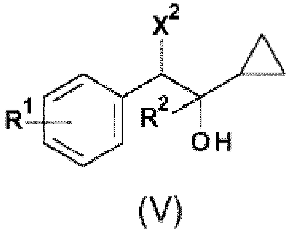
R¹ représentant chlore, brome, iode, alcoxy, amino, cyano, nitro, C₁-C₄-alkyle ou phényle non substitué ou substitué par s radicaux du groupe constitué par chlore, fluor, méthoxy et éthoxy,
R₂ représentant C₁-C₄-alkyle ou phényle non substitué ou substitué par s radicaux du groupe constitué par chlore, fluor, méthoxy et éthoxy,
s étant 1, 2 ou 3,
X² étant chlore, brome ou iode.

8. Composé selon la revendication 7, dans lequel X² est brome, R¹ est 4-chloro et R₂ est méthyle.

9. Composé de formule (VI), 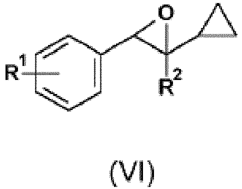
R¹ représentant chlore, brome, iode, alcoxy, amino, cyano, nitro, C₁-C₄-alkyle ou phényle non substitué ou substitué par s radicaux du groupe constitué par chlore, fluor, méthoxy et éthoxy,
R₂ représentant C₁-C₄-alkyle ou phényle non substitué ou substitué par s radicaux du groupe constitué par chlore, fluor, méthoxy et éthoxy,
s étant 1, 2 ou 3.

10. Composé selon la revendication 9, dans lequel R¹ est 4-chloro et R₂ est méthyle.
